# EUROPÄISCHE PATENTSCHRIFT

(11) **EP 0 618 921 B1**
(45) Veröffentlichungstag und Bekanntmachung des Hinweises auf die Patenterteilung: **08.01.1997**
(21) Anmeldenummer: 92923395.5
(22) Anmeldetag: 13.11.1992
(51) Int. Cl.: C07H 15/04

(54) **VERFAHREN ZUR HERSTELLUNG VON ALKYLOLIGOGLUCOSIDEN MIT VERMINDERTEM POLYGLUCOSEGEHALT**
PROCESS FOR PREPARING ALKYLOLIGOGLUCOSIDES HAVING A LOWER POLYGLUCOSE CONTENT
PROCEDE DE PREPARATION D'OLIGOGLUCOSIDES D'ALKYLE A TENEUR REDUITE EN POLYGLUCOSE

(30) Priorität: 21.11.1991 DE 4138250
(43) Veröffentlichungstag der Anmeldung: 12.10.1994
(73) Patentinhaber: Henkel Kommanditgesellschaft auf Aktien, 40191 Düsseldorf (DE)
(72) Erfinder: SCHULZ, Paul, D-5600 Wuppertal 1 (DE); ESKUCHEN, Rainer, D-4000 Düsseldorf 13 (DE)
(86) Internationale Anmeldenummer: EP9202607
(87) Internationale Veröffentlichungsnummer: WO9310133

(56) Entgegenhaltungen:
- EP-A- 0 301 298
- EP-A- 0 306 651
- EP-A- 0 306 652
- EP-A- 0 377 831
- EP-A- 0 377 883
- EP-A- 0 501 032

## Beschreibung

### Gebiet der Erfindung

Die Erfindung betrifft ein Verfahren zur Herstellung von Alkyloligoglucosiden mit vermindertem Polyglucosegehalt, bei dem man zunächst Glucosesirup in Gegenwart von sauren Katalysatoren mit Niedrigalkoholen umsetzt, Wasser azeotrop abdestilliert und die resultierende Mischung aus Glucosid und Niedrigalkohol anschließend einer Umacetalisierung mit einem Fettalkohol unterwirft.

### Stand der Technik

Oberflächenaktive Alkyloligoglucoside sind als Rohstoffe für die Herstellung von Waschmitteln bereits seit langem bekannt. Ihre Herstellung erfolgt üblicherweise durch säurekatalysierte Acetalisierung von Glucose mit langkettigen Fettalkoholen. Bei den als "Umacetalisierungsverfahren" oder "Butanol-Route" bekannten Verfahren wird im ersten Schritt die Glucose mit Butanol zum Butylglucosid umgesetzt, welches dann in einer zweiten Stufe einer Umacetalisierung mit einem Fettalkohol unterworfen wird.

Aus der Europäischen Patentanmeldung **EP 0 319 616 A1** ist ein Verfahren zur Herstellung von Niedrigalkyloligoglykosiden bekannt, bei dem man eine wäßrige Zuckerlösung mit einem kurzkettigen Alkohol vermischt, der homogenen Lösung einen sauren Katalysator zusetzt und die Mischung anschließend bei 60 bis 200, vorzugsweise 80 bis 150°C zur Reaktion bringt, wobei enthaltenes Wasser azeotrop abdestilliert wird.

Gemäß der Lehre der Europäischen Patentanmeldung **EP 0 301 298** A1 lassen sich langkettige Alkyloligoglykoside erhalten, indem man eine Mischung aus Butanol und einem sauren Katalysator auf Rückflußtemperatur erhitzt, eine wäßrige Glykoselösung in das System eindosiert, Wasser als azeotropes Gemisch mit Butanol aus dem Gleichgewicht entfernt, anschließend den Fettalkohol zusetzt und gleichzeitig freiwerdendes Butanol abdestilliert.

Die Europäische Patentanmeldung **EP 0 377 831 A1** beschreibt ein weiteres Verfahren zur Herstellung von langkettigen Alkyloligoglykosiden, bei dem man eine saure Mischung eines Butylglykosids in Butanol bei 100 bis 125°C mit Fettalkoholen umsetzt.

Die Internationale Patentanmeldung **WO 89/00923** beschreibt ebenfalls ein Verfahren zur Herstellung von langkettigen Alkyloligoglykosiden, bei dem man Glykose in Gegenwart eines sauren Katalysators mit einem wäßrigen kurzkettigen Alkohol bei Temperaturen oberhalb von 100°C und einem Druck oberhalb 1 bar zur Reaktion bringt und das resultierende fließfähige Zwischenprodukt unter solchen Bedingungen mit dem Fettalkohol umacetalisiert, daß das Wasser und der kurzkettige Alkohol unmittelbar in die Dampfphase übergehen und somit dem Gleichgewicht entzogen werden.

Die Europäischen Patentschrift **EP 0 252 241 B1** beschreibt schließlich ein Verfahren, bei dem man Butylglykoside mit wäßrigem Glykosesirup umsetzt und das Wasser azeotrop aus der Reaktionsmischung entfernt.

Ein wesentlicher Gesichtspunkt bei der Herstellung von Alkyloligoglucosiden ist die Tatsache, daß unter den Bedingungen der Acetalisierung nicht nur ein Zuckermolekül mit einem Alkoholmolekül zusammentritt. Im Sinne einer Parallelreaktion bzw. Folgereaktion findet vielmehr auch die Bildung von Polyzuckern statt, die einen Oligomerisierungsgrad (DP-Grad) von 2 bis ca. 10 und darüber hinaus aufweisen und ebenfalls mit Fettalkoholen abreagieren können. Aus anwendungstechnischen Gründen sind weder Polyzucker noch Alkyloligoglykoside mit höheren DP-Graden in der Reaktionsmischung erwünscht. Ein besonderes Ziel bei der Herstellung von Alkyloligoglucosiden besteht daher üblicherweise darin, die Bildung dieser unerwünschten Nebenprodukte, insbesondere der Polyglucose, zu minimieren.

Die Aufgabe der Erfindung bestand nun darin, ein verbessertes Verfahren zu entwickeln, nach dem Alkyloligoglucoside mit einem gegenüber dem Stand der Technik verminderten Gehalt an Polyglucose zugänglich sind.

### Beschreibung der Erfindung

Gegenstand der Erfindung ist ein Verfahren zur Herstellung von Alkyloligoglucosiden mit vermindertem Polyglucosegehalt, das sich dadurch auszeichnet, daß man
a) wäßrigen Glucosesirup bei einer Temperatur von 100 bis 115°C einer Mischung aus einem Niedrigalkohol und einem sauren Katalysator zudosiert,
b) im Reaktionsgemisch enthaltenes sowie freigesetztes Wasser azeotrop abdestilliert,
c) die Reaktionsmischung nach Beendigung der Zugabe an Glucosesirup bei einer Temperatur von 100 bis 115°C einer Nachreaktion unterwirft,
d) das als Zwischenprodukt gebildete Niedrigalkyloligoglucosid/Niedrigalkohol-Gemisch bei einer Temperatur von 110 bis 125°C einem Fettalkohol zudosiert,
e) im Reaktionsgemisch enthaltenen sowie freigesetzten Niedrigalkohol abdestilliert,
f) die Reaktionsmischung nach Beendigung der Zugabe an Niedrigalkyloligoglucosid/Niedrigalkohol-Gemisch bei einer Temperatur von 105 bis 115°C einer weiteren Nachreaktion unterwirft sowie
g) die Reaktionsmischung anschließend in an sich bekannter Weise neutralisiert und von überschüssigem Fettalkohol befreit.

Überraschenderweise wurde gefunden, das sich Alkyloligoglucoside mit einem signifikant verminderten Gehalt an Polyglucose erhalten lassen, wenn man die Alkoholyse und die Umacetalisierung sowie die sich an die letzte Stufe anschließende Nachreaktion in jeweils speziellen Temperaturintervallen durchführt. Die Erfindung schließt ferner die Erkenntnis ein, daß sich die Temperaturbelastung der Reaktionsmischung verringern und die Farbqualität der Reaktionsprodukte verbessern läßt, wenn man die Verdampfung der flüchtigen Reaktionskomponente, die die Verschiebung des Gleichgewichtes bewirkt - also Wasser in der ersten, Niedrigalkohol in der zweiten Stufe - nicht ausschließlich aus dem Reaktionsgefäß, sondern vorzugsweise über einen Fallfilmverdampfer durchführt, durch den die Reaktionsmischung im Kreis gepumpt wird.

Unter **Glucosesirup** ist ein hochabgebautes wäßriges Stärkeprodukt zu verstehen, das einem Feststoffanteil von 50 bis 85, vorzugsweise 70 bis 80 Gew.-% - bezogen auf den Sirup - sowie einen DP-Grad 1 (d.h. einen monomeren Glucoseanteil) von 90 bis 100 Gew.-% - bezogen auf den Feststoffanteil - aufweist.

Unter **Niedrigalkohole** sind im Sinne des erfindungsgemäßen Verfahrens primäre Alkohol der Formel (I) zu verstehen,

R¹-OH (I)

in der R¹ für einen linearen oder verzweigten Alkylrest mit 1 bis 8 Kohlenstoffatomen steht. Typische Beispiele sind Methanol, Ethanol, n-Propanol, Isopropylalkohol, i-Butanol, sec.-Butanol, tert.-Butanol, Pentanol, Hexanol, Heptanol, n-Octanol und 2-Ethylhexanol. Vorzugsweise wird n-Butanol eingesetzt.

Die Acetalisierung wird in Gegenwart **saurer Katalysatoren** durchgeführt. Typische Beispiel hierfür sind Methansulfonsäure, Butansulfonsäure und Sulfobernsteinsäure. Bevorzugt wird p-Toluolsulfonsäure eingesetzt.

Zur Verlagerung des Acetalisierungsgleichgewichtes auf die Seite der Glucoside empfiehlt es sich, in der ersten Stufe des erfindungsgemäßen Verfahrens den Niedrigalkohol in erheblichen Überschuß vorzulegen. Typischerweise können Glucose und Niedrigalkohol in einem molaren Verhältnis von 1 : 5 bis 1 : 10, vorzugsweise 1 : 6 bis 1 : 8 eingesetzt werden.

Die Einsatzmenge des sauren Katalysators kann 3 ∗ 10⁻³ bis 2 ∗ 10⁻², vorzugsweise 5 ∗ 10⁻³ bis 1 ∗ 10⁻² mol pro mol Glucose betragen.

Die Alkoholyse der Glucose stellt vorzugsweise eine Butanolyse dar. Dabei empfiehlt es sich, zunächst eine Lösung des sauren Katalysators im überschüssigen Niedrigalkohol herzustellen, die Mischung auf die Reaktionstemperatur von 100 bis 115, vorzugsweise 108 bis 113 °C zu erhitzen und den Glucosesirup kontinuierlich über einen Zeitraum von 0,1 bis 5 h zuzudosieren. In einer bevorzugten Ausführungsform des erfindungsgemäßen Verfahrens wird die Mischung aus Glucosesirup, Niedrigalkohol und Katalysator im Kreislauf über einen Fallfilmverdampfer geführt. Die hohe Stoffaustauschfläche, die geringe Filmdicke und die große Durchflußgeschwindigkeit gewährleisten eine besonders schonende Thermostatisierung der Reaktionsmischung, was sich in einer hohen Farbqualität der Reaktionsendprodukte niederschlägt.

Zur Einstellung des Gleichgewichtes ist es erforderlich, sowohl das Wasser aus dem Glucosesirup, als auch das Reaktionswasser möglichst rasch und möglichst vollständig aus der Reaktionsmischung zu entfernen. Dies geschiet vorzugsweise vermittels einer handelsüblichen Destillationskolonne, über die ein azeotropes Gemisch aus Wasser und Niedrigalkohol abgezogen werden kann. Im Fall von Butanol als Niedrigalkohol bildet sich ein Azeotrop mit einem Siedepunkt von ca. 93°C, das besonders leicht abdestilliert werden kann.

Das über die Kolonne abgetrennte Alkohol/Wasser-Gemisch kann in einem Abscheidebehälter in eine obere alkoholreiche Phase, die in die Kolonne zurückgeführt wird sowie eine untere alkoholarme Phase getrennt werden, die einer getrennten Aufarbeitung zugeführt wird. Auf diese Weise ist gewährleistet, daß das Verfahren mit einem geringen Verlust an Niedrigalkohol auskommt.

An die Zugabe des Glucosesirups schließt sich eine Nachreaktion bei 100 bis 115°C über einen Zeitraum von ca. 0,1 bis 2, vorzugsweise 0,5 bis 1,5 h an, während der sichergestellt wird, daß die Glucose vollständig, d. h. zu mindestens 99 Gew.-% - bezogen auf die Einsatzmenge - abreagiert. Das Reaktionsende kann auch durch den Anstieg der Temperatur in der Destillationskolonne erkannt werden, sobald anstelle des Wasser/Butanolazeotrops nur noch reines Butanol verdampft wird.

Nach der Alkoholyse wird das resultierende Gemisch aus Niedrigalkylglucosid und Niedrigalkohol in die 2. Stufe, die Umacetalisierung überführt.

Zur Umacetalisierung kommen **Fettalkohole** der Formel (II) in Betracht,

R²-OH (II)

in der R² für einen linearen oder verzweigten Alkylrest mit 10 bis 22 Kohlenstoffatomen steht. Typische Beispiele sind Caprinalkohol, Myristylalkohol, Laurylalkohol, Cetylalkohol, Stearylalkohol, Arachylalkohol und Behenylalkohol sowie deren Gemische.

Wie in der Fettchemie üblich, können die Fettalkohole auch in Form technischer Gemische vorliegen, wie sie z. B. durch Hochdruckhydrierung von Methylestern auf Basis pflanzlicher oder tierischer Öle und Fette, beispielsweise Palmöl, Palmkernöl, Kokosöl oder Rindertalg erhalten werden. Herstellungsbedingt können diese Fettalkohole auch geringe Anteile ungesättigter Verbindungen enthalten. Bevorzugt sind Fettalkohole einer Iodzahl von 0 bis 20, vorzugsweise 0 bis 5. Eine weitere Gruppe technischer geeigneter Fettalkohole stellen die Oxoalkohole des angegebenen Kohlenstoffzahlbereichs dar, die durch Hydrierung von Aldehyden aus der Roelen'schen Oxoreaktion gewonnen werden und einen Anteil von 5 bis 25 Gew.-% verzweigter Species enthalten können. Bevorzugt ist der Einsatz von technischen Kokosfettalkoholschnitten mit 12 bis 18, vorzugsweise 12 bis 14 Kohlenstoffatomen.

Wie schon in der Alkoholyse, ist es auch in der Umacetalisierung von Vorteil, die Alkoholkomponente in großem Überschuß vorzulegen. Das molare Verhältnis von Glucose und Fettalkohol kann dabei 1 : 4 bis 1 : 6, vorzugsweise 1 : 4,5 bis 1 : 5 betragen.

Zur Durchführung der Umacetalisierung empfiehlt es sich, den Fettalkohol vorzulegen, auf die Reaktionstemperatur von 110 bis 125, vorzugsweise 115 bis 120°C zu erhitzen und danach das Niedrigalkoligoglucosid/Niedrigalkohol-Gemisch aus der Alkoholyse-Stufe über einen Zeitraum von 0,1 bis 5, vorzugsweise 1 bis 3 h zuzudosieren. In einer bevorzugten Ausführungsform des erfindungsgemäßen Verfahrens erfolgt auch hier wieder die Entfernung der leichsiedenden Reaktionskomponente mit Hilfe eines Fallfilmverdampfers, durch den die Reaktionsmischung im Kreislauf gepumpt wird. Weiterhin hat es sich als vorteilhaft erwiesen, die Reaktion unter einem verminderten Druck von 5 bis 100 mbar durchzuführen.

Ferner empfiehlt es sich, freien Niedrigalkohol sowie Niedrigalkohol, der bei der Umacetalisierung freigesetzt wird, umgehend aus dem Gleichgewicht zu entfernen. Wie bei der Alkoholyse bedient man sich hierfür vorteilhafterweise einer handelsüblichen Destillationskolonne. Um aufsteigende Fettalkoholdämpfe abzutreiben und einem Verlust an Fettalkohol vorzubeugen, hat es sich als optimal erwiesen, einen Teil des abdestillierten Niedrigalkohols erneut auf den Kolonnenkopf aufzugeben, wobei ein Rücklaufverhältnis von 0,1 bis 0,2 bevorzugt ist.

Nach Beendigung der Zugabe des Niedrigalkyloligoglucosid/ Niedrigalkohol-Gemischs wird das Reaktionsprodukt einer weiteren Nachreaktion bei einer gegenüber der Umacetalisierung verminderten Temperatur von 105 bis 115, vorzugsweise 108 bis 112°C über einen Zeitraum von 0,1 bis 5, vorzugsweise 1 bis 3 h unterworfen. Vorzugsweise liegt die Temperatur in der Nachreaktion 8 bis 10°C unter der Temperatur der Umacetalisierung. Im Verlauf der Nachreaktion empfiehlt es sich ferner, den Anlagendruck schrittweise auf 10 bis 20 mbar abzusenken. Zur leichteren Abtrennung des restlichen Niedrigalkohols können Destillationskolonne und/oder Fallfilmverdampfer in Gleichstromrichtung mit einem leichten Stickstoffstrom beaufschlagt werden.

Nach dem Abschluß der Reaktion kann die Reaktionsmischung in an sich bekannter Weise mit Natriumhydroxidlösung und/oder Magnesiumoxid neutralisiert werden. Die Aufarbeitung, insbesondere die Abtrennung des überschüssigen Fettalkohols, kann dabei nach den Verfahren des Stands der Technik erfolgen.

Neben der zweistufigen diskontinuierlichen Arbeitsweise kann das erfindungsgemäße Verfahren auch kontinuierlich durchgeführt werden. So ist es beispielsweise möglich, die Alkoholyse des Glucosesirups in einer Rührkesselkaskade bestehend aus Rührreaktoren mit Fallfilmverdampfer und Destillationskolonne durchzuführen.

In einem solchen Fall wird die Reaktion der Glucose zum Niedrigalkyloligoglucosid bei einer Temperatur von vorzugsweise 100 bis 112°C und einer Verweilzeit von vorzugsweise 2 bis 3 h erreicht, indem man Niedrigalkohol, Katalysator und Glucosesirup kontinuierlich in den ersten Reaktor einer Kaskade aus zwei bis drei Rührkesseln eindosiert; die Nachreaktion kann in einem der weiteren Rührkessel bei 110 bis 112°C über einen Zeitraum von ca. 1 h durchgeführt werden.

Die Umacetalisierung des Niedrigalkyloligoglucosids zum langkettigen Alkyloligoglucosid kann in einer ähnlichen Rührkesselkaskade durchgeführt werden. Hierbei empfiehlt es sich, das Alkohol/Glucosidgemisch und den Fettalkohol kontinuierlich bei einer Temperatur von 115 bis 125°C, einem verminderten Druck von ca. 100 mbar und einer Verweilzeit von ca. 2 bis 3 h in die Kaskade einzudosieren. Die Nachreaktion kann in einem weiteren Kessel bei einer Temperatur von 110 bis 115°C, einem Druck von 5 bis 10 mbar und einer Verweilzeit von ca. 1 h erfolgen, während der Niedrigalkohol über die Destillationskolonnen auf den Rührreaktoren abgetrennt wird.

### Gewerbliche Anwendbarkeit

Die nach dem erfindungsgemäßen Verfahren hergestellten Alkyloligoglucoside weisen einen Polyglucosegehalt von weniger als 5 Gew.-% - bezogen auf den Feststoffgehalt des Produktes auf - und zeichnen sich durch gute Farbqualität aus.

Sie eignen sich als Rohstoffe zur Herstellung von Wasch-, Spül- und Reinigungsmitteln sowie Produkten zur Haar- und Körperpflege, in denen sie in Mengen von 0,1 bis 50, vorzugsweise 1 bis 25 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

a) **Stufe 1 (Butanolyse)**
   In einem 1000-1-Rührkessel mit Mantelheizung wurde eine Mischung von 592 kg n-Butanol (8000 Mol) und 1,9 kg - entsprechend 1 * 10⁻ mol pro mol Glucose - p-Toluolsulfonsäure vorgelegt. Über eine Kreiselpumpe und einen Fallfilmverdampfer wurde die Mischung im Kreis gepumpt und unter Beaufschlagung mit Stickstoff auf eine Temperatur von 112°C erhitzt. Innerhalb von 3 h wurde der Mischung 257 kg Glucosesirup (DP-Grad 1 = 95 Gew.-%, Wasseranteil = 30 Gew.-%), entsprechend einer Menge von 180 kg (1000 Mol) Glucose, zudosiert.
   Um den Wassergehalt im Reaktionsgemisch möglichst gering, d. h. kleiner 2 Ge.-% bezogen auf die Mischung, zu halten und die Menge an unerwünschten Nebenprodukte zu minimieren, wurde das Wasser aus dem Reaktionsansatz über eine Destillationskolonne als Butanol/Wasser-Azeotrop entfernt. Das am Kopf der Kolonne anfallende Destillat wurde in einen Abscheider geleitet und die butanolreiche obere Phase in die Kolonne zurückgeführt, während die wasserreiche Phase getrennt aufgearbeitet wurde.
   Nach Beendigung der Zugabe von Glucosesirup wurde die Reaktionsmischung bei 112°C einer weiteren Nachreaktion von ca. 1 h unterworfen.
b) **Stufe 2 (Umacetalisierung)**
   In einem 2500-1-Rührkessel mit Mantelheizung wurden ca. 870 kg (4500 Mol) C12/14-Kokosfettalkohol (Lorol^{(R)} Spezial, Hydroxylzahl 290, Fa.Henkel KGaA, Düsseldorf, FRG) - entsprechend einem molaren Einsatzverhältnis von Glucose zu Fettalkohol von 1 : 4,5 - vorgelegt. Über eine Kreiselpumpe und einen Fallfilmverdampfer wurde die Mischung im Kreis gepumpt und unter Beaufschlagung mit Stickstoff auf eine Temperatur von 125°C erhitzt. Am Reaktor wurde ein verminderter Druck von ca. 100 mbar eingestellt und über einen Zeitraum von 3 h ca. 900 kg des Reaktionsproduktes der Stufe 1 (entsprechend ca. 216 g Butylglucosid und ca. 525 kg n-Butanol) eindosiert.
   Freigesetztes sowie in der Reaktionsmischung enthaltenes Butanol wurde über eine Destillationskolonne aus der Reaktionsmischung entfernt, wobei ein Teil des Butanols (Rücklaufverhältnis 0,1) in die Kolonne zurückgeführt wurde, um aufsteigende Fettalkoholdämpfe abzutreiben.
   Nach Beendigung der Zugabe des Butylglucosid/Butanol-Gemischs wurde die Reaktionsmischung bei 110°C einer Nachreaktion von ca. 1 h unterworfen, wobei der Druck im Reaktor schrittweise auf 10 mbar abgesenkt wurde. Zur besseren Abtrennung des Butanols wurde der Fallfilmverdampfer im Gleichstrom mit Stickstoff beaufschlagt.
   Nach Beendigung der Butanolabtrennung wurde die Reaktionsmischung mit wäßriger Natriumhydroxyidlösung auf pH 9 eingestellt und mit 0,05 Gew.-% Magnesiumoxid - bezogen auf die Mischung - versetzt. Der Gehalt an Polyglucose betrug 4,7 Gew.-% bezogen auf den Feststoffanteil des Produktes.
   Die anschließende destillative Abtrennung des Fettalkohols erfolgte in an sich bekannter Weise nach den Verfahren des Stands der Technik.

### Vergleichsbeispiel 1:

Beispiel 1 wurde wiederholt, die Nachreaktion in der Umacetalisierungs-Stufe jedoch bei 120°C durchgeführt. Der Gehalt an Polyglucose betrug 6,1 Gew.-% bezogen auf den Feststoffanteil des Produktes.

## Patentansprüche

1. Verfahren zur Herstellung von Alkyloligoglucosiden mit vermindertem Polyglucosegehalt, **dadurch gekennzeichnet**, daß man
a) wäßrigen Glucosesirup bei einer Temperatur von 100 bis 115°C einer Mischung aus einem Niedrigalkohol und einem sauren Katalysator zudosiert,
b) im Reaktionsgemisch enthaltenes sowie freigesetztes Wasser azeotrop abdestilliert,
c) die Reaktionsmischung nach Beendigung der Zugabe an Glucosesirup bei einer Temperatur von 100 bis 115°C einer Nachreaktion unterwirft,
d) das als Zwischenprodukt gebildete Niedrigalkyloligoglucosid/Niedrigalkohol-Gemisch bei einer Temperatur von 110 bis 125°C einem Fettalkohol zudosiert,
e) im Reaktionsgemisch enthaltenen sowie freigesetzten Niedrigalkohol abdestilliert,
f) die Reaktionsmischung nach Beendigung der Zugabe an Niedrigalkyloligoglucosid/Niedrigalkohol-Gemisch bei einer Temperatur von 105 bis 115°C einer weiteren Nachreaktion unterwirft sowie
g) die Reaktionsmischung anschließend in an sich bekannter Weise neutralisiert und von überschüssigem Fettalkohol befreit.

2. Verfahren nach Anspruch 1, **dadurch gekennzeichnet**, daß man Glucosesirup mit einem Feststoffanteil von 50 bis 85 Gew.-% - bezogen auf den Sirup - einsetzt.

3. Verfahren nach den Ansprüchen 1 und 2, **dadurch gekennzeichnet**, daß man Glucosesirup mit einem DP-Grad 1 von 90 bis 100 Gew.-% - bezogen auf den Sirup - einsetzt.

4. Verfahren nach mindestens einem der Ansprüche 1 bis 3, **dadurch gekennzeichnet**, daß man Niedrigalkohole der Formel (I) einsetzt,
R¹-OH (I)
in der R¹ für einen linearen oder verzweigten Alkylrest mit 1 bis 8 Kohlenstoffatomen steht.

5. Verfahren nach mindestens einem der Ansprüche 1 bis 4, **dadurch gekennzeichnet**, daß man saure Katalysatoren einsetzt, die ausgewählt sind aus der Gruppe, die von p-Toluolsulfonsäure, Methansulfonsäure, Butansulfonsäure und Sulfobernsteinsäure gebildet wird.

6. Verfahren nach mindestens einem der Ansprüche 1 bis 5, **dadurch gekennzeichnet**, daß man Glucose und Niedrigalkohol in einem molaren Verhältnis von 1 : 5 bis 1 : 10 einsetzt.

7. Verfahren nach mindestens einem der Ansprüche 1 bis 6, **dadurch gekennzeichnet**, daß man den sauren Katalysator in Mengen von 3 * 10⁻³ bis 2 * 10⁻² mol pro mol Glucose einsetzt.

8. Verfahren nach mindestens einem der Ansprüche 1 bis 7, **dadurch gekennzeichnet**, daß man die Mischung aus Glucosesirup, Niedrigalkohol und Katalysator im Kreislauf über einen Fallfilmverdampfer als Wärmeaustauscher führt.

9. Verfahren nach mindestens einem der Ansprüche 1 bis 8, **dadurch gekennzeichnet**, daß man Fettalkohole der Formel (II) einsetzt,
R²-OH (II)
in der R² für einen linearen oder verzweigten Alkylrest mit 10 bis 22 Kohlenstoffatomen steht.

10. Verfahren nach mindestens einem der Ansprüche 1 bis 9, **dadurch gekennzeichnet**, daß man Glucose und Fettalkohol im molaren Verhältnis von 1 : 4 bis 1 : 6 einsetzt.

11. Verfahren nach mindestens einem der Ansprüche 1 bis 10, **dadurch gekennzeichnet**, daß man die Umsetzung des Niedrigalkyloligoglucosid/Niedrigalkohol-Gemischs mit dem Fettalkohol unter vermindertem Druck durchführt.

12. Verfahren nach mindestens einem der Ansprüche 1 bis 11, **dadurch gekennzeichnet**, daß man das Verfahren kontinuierlich in einer Rührkesselkaskade durchführt.

## Claims

1. A process for the production of alkyl oligoglucosides of reduced polyglucose content, **characterized in that**
a) water-containing glucose sirup is added to a mixture of a lower alcohol and an acidic catalyst at a temperature of 100 to 115°C,
b) water present in the reaction mixture and water which has been released are azeotropically distilled off,
c) after the glucose sirup has been added, the reaction mixture is subjected to an after-reaction at a temperature of 100 to 115°C,
d) the lower alkyl oligoglucoside/lower alcohol mixture formed as intermediate product is added to a fatty alcohol at a temperature of 110 to 125°C,
e) lower alcohol present in the reaction mixture and lower alcohol which has been released are distilled off,
f) after the lower alkyl oligoglucoside/lower alcohol mixture has been added, the reaction mixture is subjected to another after-reaction at a temperature of 105 to 115°C and
g) the reaction mixture is subsequently neutralized by methods known per se and freed from excess fatty alcohol.

2. A process as claimed in claim 1, **characterized in** **that** glucose sirup having a solids content of 50 to 85% by weight, based on the sirup, is used.

3. A process as claimed in claims 1 and 2, **characterized in that** glucose sirup having a DP degree 1 of 90 to 100% by weight, based on the sirup, is used.

4. A process as claimed in at least one of claims 1 to 3, **characterized in that** lower alcohols corresponding to formula (I):
R¹-OH (I)
in which R¹ is a linear or branched alkyl radical containing 1 to 8 carbon atoms,
are used.

5. A process as claimed in at least one of claims 1 to 4, **characterized in that** acidic catalysts selected from the group consisting of p-toluenesulfonic acid, methanesulfonic acid, butanesulfonic acid and sulfosuccinic acid are used.

6. A process as claimed in at least one of claims 1 to 5, **characterized in that** glucose and lower alcohol are used in a molar ratio of 1:5 to 1:10.

7. A process as claimed in at least one of claims 1 to 6, **characterized in that** the acidic catalyst is used in quantities of 3·10⁻³ to 2·10⁻² moles per mole of glucose.

8. A process as claimed in at least one of claims 1 to 7, **characterized in that** the mixture of glucose sirup, lower alcohol and catalyst is circulated through a falling-film evaporator as heat exchanger.

9. A process as claimed in at least one of claims 1 to 8, **characterized in that** fatty alcohols corresponding to formula (II):
R²-OH (II)
in which R² is a linear or branched alkyl radical containing 10 to 22 carbon atoms,
are used.

10. A process as claimed in at least one of claims 1 to 9, **characterized in that** glucose and fatty alcohol are used in a molar ratio of 1:4 to 1:6.

11. A process as claimed in at least one of claims 1 to 10, **characterized in that** the reaction of the lower alkyl oligoglucoside/lower alcohol mixture with the fatty alcohol is carried out under reduced pressure.

12. A process as claimed in at least one of claims 1 to 11, **characterized in that** the process is carried out continuously in a cascade of stirred reactors.

## Revendications

1. Procédé de préparation d'alkyloligoglucosides à teneur réduite en polyglucose,
caractérisé en ce que
a) on ajoute progressivement du sirop aqueux de glucose à une température de 100 à 115°C à un mélange d'alcool inférieur et de catalyseur acide,
b) on élimine par distillation azéotropique l'eau contenue ainsi que celle libérée dans le mélange réactionnel,
c) on soumet à une postréaction à une température de 100 à 115°C le mélange réactionnel après l'addition au sirop de glucose,
d) on ajoute progressivement le mélange formé en intermédiaire d'(alkyle inférieur)oligoglucoside/alcool inférieur à une température de 110 à 125°C à un alcool gras,
e) on élimine par distillation l'alcool inférieur contenu ainsi que celui libéré dans le mélange réactionnel,
f) on soumet le mélange réactionnel après l'addition du mélange d'(alkyle inférieur)oligoglucoside/alcool inférieur à une température de 105 à 115°C à une autre postréaction ainsi que,
g) on neutralise ensuite le mélange réactionnel de la manière connue et on élimine l'alcool gras en excès.

2. Procédé selon la revendication 1,
caractérisé en ce qu'
on utilise du sirop de glucose avec une teneur en solide de 50 à 85 % en poids, par rapport au sirop.

3. Procédé selon les revendications 1 et 2,
caractérisé en ce qu'
on utilise du sirop de glucose avec un degré DP 1 de 90 à 100 % en poids, par rapport au sirop.

4. Procédé selon au moins l'une des revendications 1 à 3,
caractérisé en ce qu'
on utilise des alcools inférieurs de formule (I),
R¹OH (I)
où R¹ représente un reste alkyle linéaire ou ramifié de 1 à 8 atomes de carbone.

5. Procédé selon au moins l'une des revendications 1 à 4,
caractérisé en ce qu'
on utilise des catalyseurs acides qu'on choisit dans le groupe formé par l'acide p-toluènesulfonique, l'acide méthanesulfonique, l'acide sulfosuccinique.

6. Procédé selon au moins l'une des revendications 1 à 5,
caractérisé en ce qu'
on utilise du glucose et un alcool inférieur en un rapport molaire de 1:5 à 1:10.

7. Procédé selon au moins l'une des revendications 1 à 6,
caractérisé en ce qu'
on utilise le catalyseur acide en des quantités de 3 * 10⁻³ à 2 * 10⁻² mol par mol de glucose.

8. Procédé selon au moins l'une des revendications 1 à 7,
caractérisé en ce qu'
on fait passer le mélange de sirop de glucose, alcool inférieur et catalyseur dans un circuit via un évaporateur à film tombant comme échangeur de chaleur.

9. Procédé selon au moins l'une des revendications 1 à 8,
caractérisé en ce qu'
on utilise des alcools gras de formule (II),
R²-OH (II)
où R² est un reste alkyle linéaire ou ramifié de 10 à 22 atomes de carbone.

10. Procédé selon au moins l'une des revendications 1 à 9,
caractérisé en ce qu'
on utilise du glucose et de l'alcool gras en rapport molaire de 1:4 à 1:6.

11. Procédé selon au moins l'une des revendications 1 à 10,
caractérisé en ce qu'
on réalise la réaction du mélange (alkyle inférieur)oligoglucoside/alcool inférieur avec un alcool gras sous pression réduite.

12. Procédé selon au moins l'une des revendications 1 à 11,
caractérisé en ce qu'
on réalise le procédé en continu dans une cascade de réacteurs avec agiteur.
